# FASCICULE DE BREVET EUROPEEN

(11) **EP 1 113 831 B1**
(45) Date de publication et mention de la délivrance du brevet: **17.11.2004**
(21) Numéro de dépôt: 99942997.0
(22) Date de dépôt: 17.09.1999
(51) Int. Cl.: A61M 5/31, A61M 5/315, A61M 5/178, A61C 5/04, A61M 5/00

(54) **SERINGUES POUR L'ADMINISTRATION DE FORMULATIONS PATEUSES OU SEMI-SOLIDES**
SPRITZEN ZUR VERABREICHUNG VON ZÄHFLÜSSIGEN ODER HALBFESTEN STOFFEN
SYRINGES FOR ADMINISTERING PASTY OR SEMI-SOLID FORMULATIONS

(30) Priorité: 18.09.1998 FR 9811699
(43) Date de publication de la demande: 11.07.2001
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHERIF CHEIKH, Roland, F-92130 Issy-les-Moulineaux (FR); AUBERT, Christophe, (St Pere De Ribes)Barcelone (ES)
(74) Mandataire: Bernasconi, Jean Raymond
(86) Numéro de dépôt international: PCT/FR1999/002218
(87) Numéro de publication internationale: WO 2000/016829

(56) Documents cités:
- EP-A- 0 397 977
- EP-A- 0 824 932
- EP-A- 0 845 275
- WO-A-94/07554
- US-A- 3 921 864
- US-A- 4 024 865
- US-A- 4 758 234
- US-A- 5 290 254
- PATENT ABSTRACTS OF JAPAN vol. 004, no. 113 (C-021), 13 août 1980 (1980-08-13) & JP 55 073352 A (TOSHIBA CORP), 3 juin 1980 (1980-06-03)

## Description

La présente invention a trait à l'administration parentérale, par injection, de formulations médicamenteuses ou autres, qui ne sont ni liquides, comme les formulations injectables classiques, ni solides comme les implants, mais pâteuses ou très visqueuses, et désignées ci-après comme des formulations semi-solides.

Il existe de nombreuses variantes de seringues à injection parentérale de liquides au moyen d'une aiguille pour l'injection parentérale. Ces seringues ne sont pratiquement pas utilisables pour l'administration parentérale de formulations semi-solides.

On connaît également divers dispositifs d'administration par voie parentérale de formulations solides telles que des implants, utilisant des trocarts de diamètres relativement importants, associés à des moyens d'expulsion permettant de faire sortir la formulation du trocart, par exemple par retrait de celui-ci. D'autres moyens d'administration utilisent des cathéters.

On connaît également des seringues destinées à l'administration de produits pâteux en dentisterie, permettant le dépôt de ciments ou autres matériaux dans les cavités dentaires. Des exemptes de telles seringues sont décrits dans les brevets US 4 121 587 et 5 603 701 qui prévoient un corps de seringue dans laquelle se déplace un piston pour expulser la masse pâteuse à travers une aiguille, en utilisant des moyens de déplacement du piston à base d'une tige filetée traversant un filetage immobile complémentaire, pour assurer une démultiplication de la force nécessaire pour l'expulsion de la masse pâteuse. Ces seringues ne sont cependant pas aptes à une injection parentérale convenable et présentent une complexité importante, qu'elles soient spécialement conçues dans toutes leurs parties ou bien qu'elles présentent des éléments agencés pour être utilisées avec des corps de seringue classique, ce qui fait qu'elles se prêtent mal à une utilisation multiple et notamment sous forme de seringue ou de carpule jetable ou non réutilisable.

Un document qui divulgue les caractéristiques du préambule de la revendication 1 et le JP 55073352.

La présente invention se propose de remédier à ces inconvénients et de fournir des seringues permettant l'injection manuelle ou automatique d'une formulation semi-solide ou pâteuse, de préférence sous un volume généralement faible , 1 ml et notamment inférieur à 1 ml ou 0,5 ml et jusqu'à quelques microlitres.

Un autre objectif de l'invention est de faciliter l'administration de formulations galéniques semi-solides, y compris de formulations à relargage contrôlé, telles que décrites notamment dans le brevet US n° 5 595 760.

Un autre objectif de l'invention est de réaliser des seringues permettant l'injection, selon les voies traditionnelles, de telles formulations, par exemple par voie sous-cutanée, intradermique, ou intramusculaire avec une grande précision aussi bien de la dose effectivement délivrée que de la position du site de dépôt.

Un autre objectif de l'invention est de permettre la délivrance, par la seringue, de formulations semi-solides grâce à des moyens d'assistance ou de démultiplication des forces.

Un autre objectif de l'invention est de réaliser ces seringues sous une forme extrêmement simple permettant une production massive à un coût très réduit.

Un autre objectif de l'invention est de permettre un préchargement extrêmement facile de la formulation semi-solide destinée à être administrée.

Un autre objectif de l'invention est de réaliser des ensembles de seringue qui permettent l'administration parentérale de volumes différents ou variables de formulation en diminuant le plus possible la nécessité de réaliser des éléments spécifiques à chaque volume de formulation.

Un autre objectif encore de l'invention est de réaliser des seringues en utilisant des matériaux ordinaires bien connus et généralement utilisés pour l'administration parentérale de médicaments, tels que du verre, du métal ou des matières plastiques telles que polyéthylène ou polypropylène, tout en assurant une résistance aux forces importantes qui doivent être déployées pour l'éjection de la formulation pâteuse à travers une aiguille de petit diamètre.

Un autre objectif de l'invention est de réaliser des seringues pour l'administration de produits semi-solides, permettant à l'opérateur de vérifier que l'extrémité de l'aiguille n'a pas pénétré dans une lumière vasculaire.

Un autre objectif encore de l'invention est de réaliser des seringues, notamment pour l'administration de formulations semi-solides, permettant d'assurer la stérilité de l'aiguille jusqu'au moment même de l'injection.

Certains ou la totalité de ces objectifs peuvent être atteints par les différentes formes de réalisation de l'invention.

L'invention a pour objet une seringue telle que définie dans la revendication 1.

Dans une première forme de réalisation, la seringue est de préférence préremplie d'une dose à délivrer entièrement, et est destinée à l'injection parentérale d'une formulation semi-solide, comprenant un élément creux formant réservoir, pour contenir la préparation semi-solide à injecter entre un piston et une embase d'une aiguille venant en contact avec une extrémité dudit élément formant réservoir, par exemple en étant introduite par une extrémité dans ledit réservoir, de façon que le piston parvienne en contact direct avec ladite embase à la fin de l'injection de la dose contenue dans ledit élément formant réservoir, ledit élément formant réservoir et ladite aiguille étant maintenus solidaires l'un de l'autre au niveau de ladite embase par une armature ou boîtier recevant ledit élément formant réservoir.

De façon préférée ladite armature ou boîtier formant l'enveloppe périphérique entoure, avec un jeu faible ou pratiquement nul, la surface extérieure dudit élément formant réservoir, de sorte que ce dernier peut être fabriqué avec une paroi mince et/ou en un matériau peu résistant, sans être détérioré par la pression élevée susceptible de se développer lors de l'injection et de la mise en compression de la formulation semi-solide.

De préférence ledit élément, ci-après désigné réservoir, est cylindrique et introduit et verrouillé à l'intérieur d'un corps creux constitué par ladite armature ou boîtier. Ainsi c'est le boîtier qui assure la protection et la résistance mécanique notamment à la pression de ladite seringue.

Dans une forme de réalisation particulièrement préférée ledit réservoir cylindrique peut être un tube creux rectiligne de diamètres interne et externe constants.

On préfère alors que le diamètre interne du réservoir soit proche ou même égal à celui de la lumière interne de l'aiguille qui prolonge le réservoir. Dans le cas où le diamètre interne du réservoir est supérieur à celui de la lumière de l'aiguille, on prévoit avantageusement, vers l'extrémité du réservoir recevant l'aiguille, un rétrécissement régulier conique ou en forme d'entonnoir. L'angle de conicité sera, par exemple, inférieur à 120°.

D'une façon générale on préfère que le réservoir cylindrique de la seringue, qui contient la formulation pâteuse à injecter, ait un diamètre aussi faible que possible en fonction du volume nécessaire.

Selon la viscosité de la formulation semi-solide et son volume on peut envisager, notamment, des diamètres internes de l'aiguille compris entre 0,2 et 1,2 à 1,5 mm. De préférence le diamètre interne du réservoir, et donc le diamètre du piston de seringue, variera également entre 0,2 et 5 mm. On pourra alors, en utilisant une course de piston de longueur maximum égale à 7 cm, injecter des volumes de 1 à 10 µl et jusqu'à 0.5 ou 1 ml. Le diamètre externe du réservoir peut être par exemple standardisé à 6 mm, ou 7, ce qui permet de prévoir les différents diamètres internes précités.

Dans le cas d'un réservoir tubulaire on peut prévoir que le réservoir est constitué de deux tubes disposés l'un dans l'autre de façon à accroître la résistance à la pression interne.

Dans une autre forme de réalisation un tel réservoir tubulaire peut être constitué de deux ou plusieurs tubes disposés l'un derrière l'autre et maintenus dans cette position par le boîtier, une telle réalisation permettant notamment de faciliter la formation de seringues permettant l'administration de volumes différents.

De façon avantageuse le boîtier peut être constitué de deux éléments dont l'un forme un corps creux dans lequel est introduit l'élément formant réservoir et dont l'autre referme le corps creux et emprisonne le réservoir, l'un des éléments laissant une ouverture pour le passage d'une tige de piston, et l'autre une ouverture pour le passage de l'aiguille.

Dans ce cas on préfère que l'ouverture dudit second élément de boîtier forme un guide pour une tige du piston de diamètre sensiblement égal au diamètre interne du réservoir.

Ce second élément peut également comporter des moyens de préhension ou appuie-doigts.

Suivant le cas l'injection peut être effectuée avec la force du pouce ou de la paume de la main en prévoyant une zone de préhension sur le premier ou le second élément de boîtier, pour permettre à la main de l'opérateur, agissant sur l'extrémité de la tige de piston, d'enfoncer le piston dans le boîtier.

En variante des moyens peuvent être agencés, sur ledit premier ou second élément, afin de démultiplier la force d'injection ou de remplacer la force manuelle par un moyen d'assistance mécanique ou motrice ou tout autre moyen moteur, par exemple à gaz, ressort ou électromécanique.

Par exemple l'ouverture dans ledit second élément peut avantageusement être filetée pour coopérer avec un filetage présenté par la tige de piston pour permettre un déplacement hélicoïdal de ladite tige.

En variante ledit second élément peut présenter un filetage périphérique sur lequel on peut visser une douille intérieurement filetée et présentant une tige de piston centrale.

Dans une forme de réalisation particulièrement intéressante d'un tel dispositif, l'assemblage dudit élément formant réservoir, avec l'aiguille, au niveau d'une embase d'aiguille, peut être réalisé sans collage, ni clipage, ni autre moyen d'assemblage positif, en assurant l'assemblage et la résistance aux forces tendant à désassembler ses pièces, par l'intermédiaire dudit boîtier, ledit boîtier étant agencé pour empêcher un écartement axial du réservoir et de l'aiguille.

Une telle réalisation peut également faciliter la séparation des éléments de la seringue par désassemblage après usage.

Le piston, qui peut être solidaire ou non d'une tige de piston, présente, de préférence, une forme qui s'adapte à l'embase de l'aiguille ou à l'extrémité du réservoir du côté de l'aiguille de façon à laisser un volume inutilisé aussi faible que possible lorsque le piston est arrivé dans sa position de fin d'injection. Dans le cas où la seringue présente une extrémité tronconique ou en entonnoir, le piston présente de façon avantageuse une forme complémentaire.

En outre, dès lors que l'on définit un diamètre constant et une longueur constante du tube réservoir et, éventuellement, d'une partie d'embase d'aiguille destinée à être serrée entre le tube réservoir et un élément de boîtier, on pourra utiliser un même boîtier pour des réservoirs prévus pour toute la gamme de doses de formulation.

Une réalisation de la seringue selon l'invention, dans laquelle l'armature ou boîtier assure les fonctions de renforcement du réservoir contre les pressions internes et/ou de serrage de l'embase d'aiguille contre le réservoir, peut être avantageusement mise à profit pour résoudre les problèmes d'étanchéité, soit en n'utilisant pas de joint, par exemple entre l'embase et le réservoir ou entre le piston et le réservoir, soit en reportant un joint d'étanchéité à une certaine distance de la formulation semi-solide, l'interstice étroit entre la paroi du réservoir et l'embase ou le piston ne provoquant, au plus, qu'une déperdition négligeable de formulation. On pourra alors avantageusement réaliser l'embase de l'aiguille et le piston dans un même matériau, et de préférence dans le même matériau que l'aiguille, par exemple en acier inoxydable, tout en assurant l'étanchéité pendant la conservation et le stockage et pendant l'injection pour préserver la formulation. Ainsi, par exemple, le contact entre la formulation et la seringue pourra être pratiquement ou totalement limité à l'acier inoxydable si tous les constituants, réservoir, embase, piston sont réalisés dans ce métal, ou en verre et acier, si le réservoir est réalisé sous forme d'un tube de verre.

De préférence l'embase de l'aiguille et le piston sont réalisés dans le même matériau, par exemple en acier inoxydable.

De préférence, pour éviter le risque d'injection dans un vaisseau, un dispositif d'injection de formulation semi-solide, tel que, notamment, un dispositif précité selon l'invention, comportera des moyens permettant de vérifier une éventuelle remontée de sang en provenance d'un vaisseau, et ceci sans avoir à tirer sur le piston comme on le fait pour le cas de formulations liquides.

Une première solution, non préférée, consistera à monter sur le dispositif une aiguille cathéter, c'est-à-dire une aiguille destinée à un passage parentéral et contenant, dans sa lumière, une seconde aiguille pour l'injection proprement dite, l'espace entre les deux aiguilles permettant une remontée de sang par capillarité jusqu'à une zone ouverte à l'extérieur. Cette solution n'est pas préférée car elle augmente le diamètre et la longueur de l'aiguille.

De façon avantageuse, le dispositif présente un passage, comprenant une zone visible par l'opérateur, en communication, avec la lumière interne de l'aiguille, permettant de faire apparaître, par capillarité ou dépression ou la pression intra-vasculaire, du sang en cas de pénétration de l'aiguille dans une lumière vasculaire.

Dans le cas où le sang peut remonter sous sa propre pression ou par capillarité, on prévoit que la lumière interne de l'aiguille soit en communication avec l'atmosphère extérieure par un trajet assurant une perte de charge telle qu'un passage de sang est permis mais que tout passage substantiel de formulation semi-solide ne puisse avoir lieu.

Dans une telle forme de réalisation on peut réaliser, dans la paroi de l'aiguille même, un trou de faible diamètre, par exemple inférieur à 0,3 mm et permettant de visualiser une remontée de sang. Cette solution n'est cependant pas préférée car difficile à réaliser.

Dans une deuxième forme de réalisation le passage pour le sang depuis l'aiguille peut passer dans une chambre prévue dans le réservoir et comporter un trajet allongé de perte de charge de préférence hélicoïdal, compris par exemple entre un filetage sur l'embase de l'aiguille et une surface complémentaire dans la paroi transparente du réservoir ou vice-versa, ce filetage aboutissant, le cas échéant, à une zone de visualisation du sang, ce filetage étant en communication, par son extrémité, directement ou par l'intermédiaire de la zone de visualisation, avec l'atmosphère extérieure par un trou de petit diamètre.

Dans une autre forme de réalisation très avantageuse, l'intérieur de l'aiguille et de la seringue est maintenu sous dépression et une remontée de sang aboutira, par différence de pression, dans une zone de visualisation, laquelle peut d'ailleurs être conçue sous forme du filetage précité ou comporter un tel filetage, une communication avec l'atmosphère n'étant alors pas prévue.

Dans une forme de réalisation avantageuse l'aiguille peut être recouverte par un capuchon, un emballage ou autre protection flexible rétractable, déformable ou repliable qui l'isole de l'extérieur et qui sera transpercé par l'aiguille au moment de l'injection, tout en s'effaçant sur au moins la majorité, et de préférence toute la longueur de l'aiguille pendant sa pénétration parentérale.

Une telle protection transperçable peut permettre le maintien de l'intérieur de la seringue sous vide. Lorsque l'opérateur appuie la seringue, munie de sa protection, contre la peau et enfonce la seringue, l'aiguille transperce la protection et pénètre dans l'épiderme sans perte sensible de vide, de sorte qu'en cas de pénétration dans une lumière vasculaire, le sang se trouvera aspiré dans l'aiguille et amené à une zone de visualisation telle que décrite ci-dessus.

Cet emballage peut être constitué, par exemple, d'un tube ou d'un sachet en matière plastique scellé autour de l'aiguille ou sur celle-ci.

Un tel emballage très fin, qui isole complètement l'aiguille de l'extérieur peut éventuellement être scellé à l'extrémité de l'aiguille, par exemple par thermosoudage, de façon à obturer totalement l'extrémité ou biseau de l'aiguille à la manière d'un bouchon, auquel cas le dispositif peut également être utilisé pour les injections liquides traditionnelles.

Dans le cas où l'on souhaite voir une éventuelle remontée de sang, remontant par capillarité, on peut prévoir que le trou reliant le passage à perte de charge à l'ambiance extérieure débouche, en fait, à l'intérieur de cet emballage, de sorte qu'aucune communication n'existe en réalité entre une atmosphère non stérile et l'intérieur de l'aiguille.

Dans le cas où l'emballage ou protection est réalisée par un tube ou un sachet extrêmement souple et donc fragile, on peut prévoir un capuchon rigide amovible de protection générale disposé par dessus cet emballage ou protection.

Une autre fonction d'un tel emballage ou protection peut être d'autoriser le maintien, dans la seringue, d'un vide pour un conditionnement sous vide de la formulation dans le réservoir sous une forme non-hydratée, l'hydratation pouvant alors être effectuée ultérieurement en enfonçant l'aiguille dans un réservoir, poche ou carpule contenant le milieu liquide d'injection, pour faire rentrer le volume souhaité de liquide dans la formulation, et assurer l'obtention de la formulation sous forme semi-solide.

De façon particulièrement préférée on pré-remplit le réservoir, avant le montage des différents éléments formant la seringue et, de façon particulièrement avantageuse, ce pré-remplissage peut être tel que le volume de formulation occupe la totalité de l'espace entre le piston et l'aiguille sans qu'il soit nécessaire de purger la seringue avant l'injection. Suivant la viscosité de la substance pharmaceutique, il peut même être nécessaire d'éviter le geste traditionnel qui consiste à retirer le piston pour créer une succion et ainsi vérifier le site d'injection. Ceci aurait pour effet de créer une cavitation dans la substance entraînant le risque d'un dépôt de la substance non-homogène. On utilise alors une tige de piston non reliée au piston.

Ce remplissage peut être effectué, suivant les types de formulation, soit directement avec la formulation semi-solide, soit avec une formulation susceptible de prendre un état semi-solide, par exemple une formulation formée à partir d'une poudre et d'un liquide permettant la formation d'une pâte semi-solide, introduits simultanément ou séquentiellement dans le réservoir.

De façon avantageuse le remplissage peut être effectué par un procédé dans lequel une buse de remplissage est connectée au niveau de l'une des extrémités du tube ou réservoir, provisoirement bouchée par le piston ou par un septum, ledit piston ou septum étant ensuite déplacé pendant le remplissage de la formulation, ledit tube ou réservoir étant ensuite bouché par la mise en place de l'embase de l'aiguille sur ladite extrémité.

Ceci permettra notamment d'utiliser le même procédé et le même dispositif de remplissage quelle que soit la formulation semi-solide.

L'invention a également trait à des procédés d'administration de formulations semi-solides par injection parentérale à l'aide d'un dispositif selon l'invention.

Dans un tel procédé on préfère attendre, après injection de l'aiguille, quelques instants avant de procéder à l'expulsion de la formulation semi-solide pour vérifier le site d'injection par absence de remontée de sang.

D'autres avantages et caractéristiques de l'invention apparaîtront à la lecture de la description suivante, faite à titre d'exemple non limitatif et se référant au dessin annexé dans lequel:
- la figure 1 représente une vue schématique en section longitudinale d'une seringue selon une première forme de réalisation de l'invention avec la tige de piston extraite;
- la figure 2 représente une vue schématique d'une pièce postérieure de boîtier et d'une tige de piston filetée selon une variante de l'invention ;
- la figure 3 représente une vue schématique en coupe d'un autre agencement d'une tige de piston et de pièce postérieure de boîtier ;
- la figure 4 représente une vue schématique en coupe d'un autre agencement de tige de piston et de pièce postérieure de boîtier pour une démultiplication ;
- la figure 5 représente une vue schématique en coupe d'un autre agencement de pièce postérieure de boîtier et de tige de piston, la tige de piston étant représentée séparée du mécanisme à levier ;
- la figure 6 représente une vue schématique en coupe d'une seringue selon une autre forme de réalisation comprenant deux éléments tubulaires consécutifs ;
- la figure 7 représente une vue schématique en coupe d'une autre forme de réalisation de l'invention comprenant deux éléments tubulaires concentriques ;
- la figure 8 représente une vue en coupe plus détaillée d'une seringue selon une forme de réalisation de l'invention ;
- la figure 9 représente une vue plus détaillée en coupe d'une autre forme de réalisation d'une telle seringue équipée d'un démultiplicateur de force optionnel ;
- la figure 10 représente une vue partielle en coupe d'une partie antérieure de seringue selon l'invention destinée à permettre une remontée de liquide sanguin pour visualisation ;
- la figure 11 représente une vue schématique d'une aiguille d'une seringue selon l'invention avec une enveloppe tubulaire ;
- la figure 12 représente une vue schématique d'une extrémité antérieure de seringue selon l'invention avec une enveloppe en forme de sachet ;
- la figure 13 représente une vue en section transversale de l'aiguille et de l'enveloppe de la figure 12 ;
- la figure 14 représente une vue schématique de l'aiguille et de l'enveloppe de la figure 12 lors de l'injection ;
- la figure 15 représente une vue schématique en coupe d'une autre forme de réalisation de l'invention avec une enveloppe de protection d'aiguille.

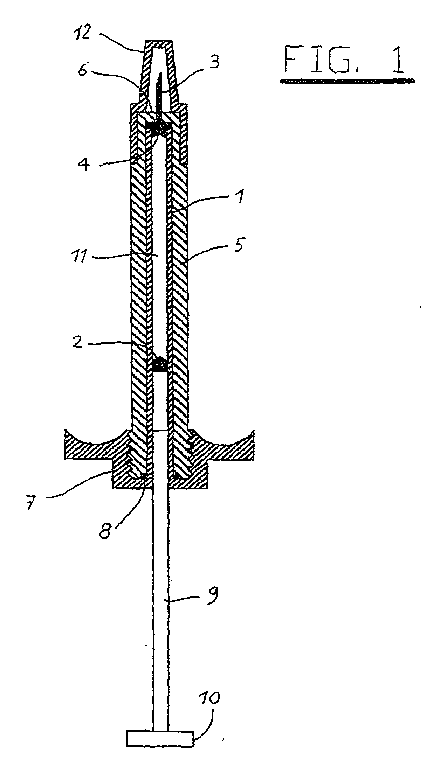

En se référant à la figure 1 on voit une seringue pour l'administration d'une formulation semi-solide comportant un élément de boîtier en forme d'un pièce tubulaire, par exemple en verre ou en matériaux plastiques, polyéthylène ou polypropylène 1, de forme cylindrique à section circulaire dans laquelle peut se déplacer un piston 2. L'aiguille 3, d'un type classique, est solidaire d'une embase 4 présentant, au niveau où l'aiguille pénètre dans l'embase, une collerette radiale suivie d'une partie cylindrique plus étroite. La lumière de l'aiguille n'a pas été représentée. L'embase 4 est enfoncée dans l'extrémité du tube réservoir 1 jusqu'à ce que sa collerette s'applique contre l'extrémité du tube. On voit que l'embase, qui est vue en section sur la figure, présente, vers le piston 2, une forme tronconique concave complémentaire de l'extrémité conique convexe du piston 2 pour favoriser l'écoulement du fluide, particulièrement s'il est visqueux pour qu'en fin d'injection le piston 2 épouse étroitement l'embase et limite ainsi les volumes morts. L'ensemble du tube réservoir 1 et de l'aiguille 3 avec son embase 4 est enfoncé dans un premier élément 5 de boîtier de forme tubulaire dont l'extrémité antérieure 6 est refermée et munie d'un passage central de diamètre supérieur à celui de l'aiguille 3. La longueur de l'élément de boîtier 5 est éventuellement telle que lorsque l'ensemble 1 + 3 est enfoncé dans l'élément 5, l'extrémité postérieure du tube 1 émerge très légèrement au-delà de l'extrémité inférieure de la pièce 5.

Le boîtier comporte un deuxième élément de boîtier 7 sous forme d'une coupelle munie d'un passage central ayant un diamètre sensiblement identique au diamètre interne du tube 1 et qui présente des pattes ou appuie-doigts latéraux. La pièce 7 est vissée par exemple sur l'élément 5 grâce à des filetages complémentaires et l'on comprend qu'à la fin du vissage l'extrémité postérieure du tube 1 est en appui contre le fond de la coupelle 7 alors que son extrémité antérieure serre la collerette de l'embase 4 contre le fond 6 de l'élément de boîtier 5, ainsi que le joint d'étanchéité 8 permettant de garantir l'asepsie de l'ensemble une fois assemblé, de sorte que l'aiguille et le réservoir sont immobilisés axialement l'un par rapport à l'autre et ne peuvent pas se déplacer.

Ce joint 8 peut être placé comme sur la figure 1, de façon à empêcher tout passage dans l'interstice entre le tube 1 et le corps 5, ou bien, au contraire, entre l'embase 4 et l'extrémité correspondante du tube 1, une asepsie de l'interstice entre le tube 1 et le corps 5 n'étant alors plus nécessaire.

Une tige de piston 9 se terminant par une surface d'appui 10 est enfoncée dans la seringue assemblée jusqu'à ce que l'extrémité antérieure de la tige 9, dont le diamètre est de préférence faiblement inférieur au diamètre intérieur du tube 1, vienne en appui contre le piston 2. Si, à partir de ce moment, on enfonce la tige 9, celle-ci repousse le piston 2 sans en être solidaire et l'on peut ainsi expulser la formulation qui est contenue dans le volume 11 situé entre le piston et l'embase.

Un capuchon 12 amovible peut être prévu pour la stérilité.

De façon avantageuse la seringue qui vient d'être décrite peut être pré-remplie. Ce pré-remplissage peut être effectué à l'aide d'une formulation qui est déjà à l'état semi-solide obtenue par exemple par mélange d'une poudre de principe actif et d'un liquide permettant la formation d'une pâte. Le remplissage peut également être effectué sous forme d'une poudre sèche qui pourrait être réhydratée à l'état pâteux de façon extemporanée avant l'injection comme décrit dans la demande de brevet français n° 96 06 886.

Le pré-remplissage s'effectue de préférence de la façon suivante. Le réservoir tubulaire 1, à l'état désassemblé, reçoit le bouchon 2 positionné dans son extrémité supérieure, c'est-à-dire l'extrémité qui va recevoir l'embase 4. Cette extrémité est placée en face d'une buse de délivrance de formulation pâteuse. La buse délivre le volume souhaité de formulation, ce qui repousse progressivement le piston 2 à l'intérieur du tube jusqu'à atteindre la position finale dans laquelle la formulation pâteuse remplit entièrement le volume 11. L'embase 4 est ensuite enfoncée dans cette extrémité et l'ensemble est assemblé dans le corps 5, 7.

Eventuellement le tube a été préalablement conditionné dans un emballage et son bouchage s'opère lors de l'introduction dans le corps creux ou boîtier qui porte l'aiguille et son capuchon, l'ensemble étant entretenu dans un second emballage.

On comprend qu'on a ainsi réalisé une seringue susceptible d'assurer le stockage puis l'injection d'une formulation semi-solide, constituée d'éléments très simple et d'un prix raisonnable par rapport aux seringues classiques. Les matériaux utilisés pourront être en plastique, en verre ou en métal. L'ensemble des éléments de la seringue est assemblé sans aucun collage ou clipage ou vissage susceptible d'entraîner des problèmes de résidus en contact avec la formulation. En outre la seringue est totalement démontable après usage.

Selon la formulation semi-solide et son volume cette seringue pourra être utilisée manuellement sans aucun mécanisme de démultiplication de force. Ainsi jusqu'à une force maximum de 50 N et préférentiellement inférieure à 30 N l'injection pourra être pratiquée manuellement en déplaçant la tige 9 par appui du pouce sur l'extrémité 10.

Si en fonction de la dureté du produit, du volume à injecter ou du diamètre de l'aiguille les forces nécessaires dépassent 30, voire 50 N, la seringue selon l'invention peut avantageusement porter un dispositif d'assistance.

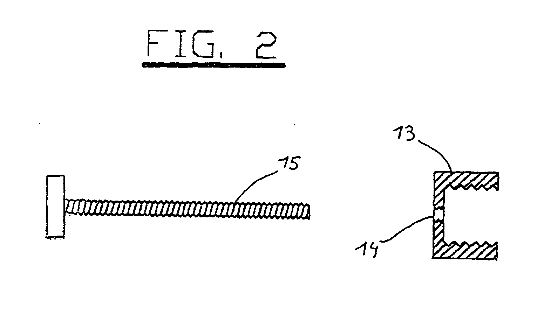

En se référant à la figure 2 on voit qu'il suffit de remplacer la pièce 7 par une pièce en forme de cupule 13 vissable de la même façon sur l'extrémité de l'élément de boîtier 5 et pourvue d'un passage taraudé 14 dans lequel peut se visser une tige de piston 15 filetée. Il suffira alors de tourner la tige filetée 15 pour provoquer son déplacement dans le tube 1 et, par conséquent, la poussée du piston 2 pour l'expulsion de la formulation. Une telle démultiplication peut facilement permettre le déploiement de forces de l'ordre de 200 N. Par ailleurs, le pas de vis permet également d'administrer une partie de la dose seulement et précisément, grâce à une graduation.

En comprend, par ailleurs, qu'à condition de réaliser les deux éléments de boîtier 5 et 7 ou 13 en un matériau suffisamment résistant, le tube 1, le piston 2 et l'embase 4 peuvent supporter des pressions très élevées sans aucune déformation sensible ni rupture.

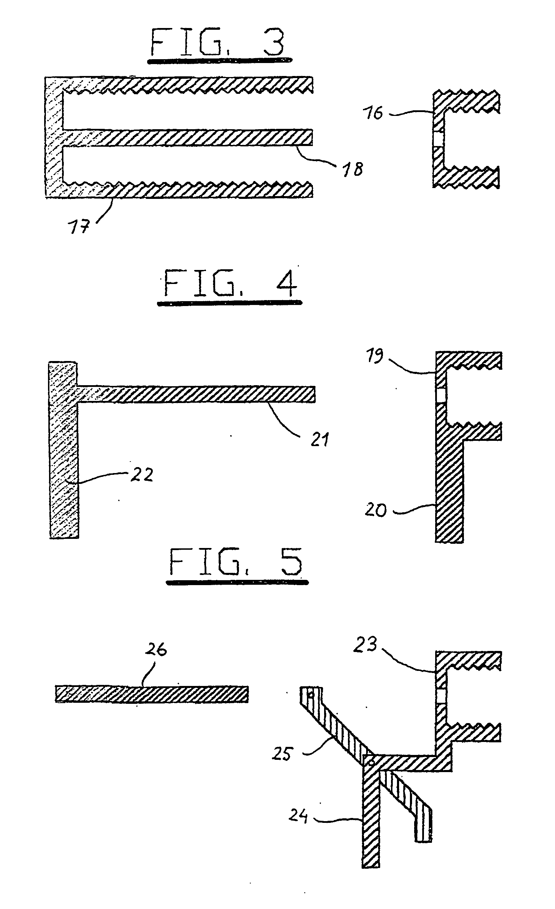

Sur la figure 3, on a représenté une autre forme de démultiplication dans laquelle le deuxième élément de boîtier 16 possède un trou non fileté et présente un filetage interne susceptible d'être vissé sur le filetage externe de l'élément de boîtier 5 et un deuxième filetage externe sur lequel peut se visser un élément en forme de douille ou manchon intérieurement fileté 17, muni à l'intérieur d'une tige cylindrique 18 faisant office de tige de piston. En vissant l'élément 17 sur le filetage externe de l'élément 16 on provoque le déplacement de l'élément 17 qui repousse le piston et assure l'injection ainsi que le dosage précis par déplacement millimétrique et graduation correspondante.

Sur la figure 4, on a représenté un dispositif dans lequel la deuxième pièce de boîtier 19, très proche de la pièce 13, présente un bras latéral 20, alors que la tige de piston 21 se termine également par un bras latéral 22, les deux bras 20, 22 pouvant être pris en charge par la main de l'utilisateur pour déployer toute la force de la main.

Sur la figure 5, on a représenté schématiquement un deuxième élément de boîtier 23 muni d'un bras latéral déporté vers l'arrière 24, sur lequel est articulé un levier 25, dont une extrémité permet la prise avec la main, simultanément à la partie 24, et dont l'autre extrémité est articulée à la tige de piston 26, de sorte que l'on peut provoquer une démultiplication, par effet de levier, de l'effort nécessaire.

La seringue représentée sur la figure 1 peut recevoir des volumes différents de formulation semi-solide qui se manifesteront par une position différente du piston 2 à la fin du préchargement de la formulation. Lorsque les volumes à injecter deviennent plus faibles encore on peut, sans modifier le boîtier 5, 7, utiliser des tubes 1 ayant le même diamètre externe mais ayant un diamètre interne plus faible, les embases 4 et pistons 2 étant alors adaptés.

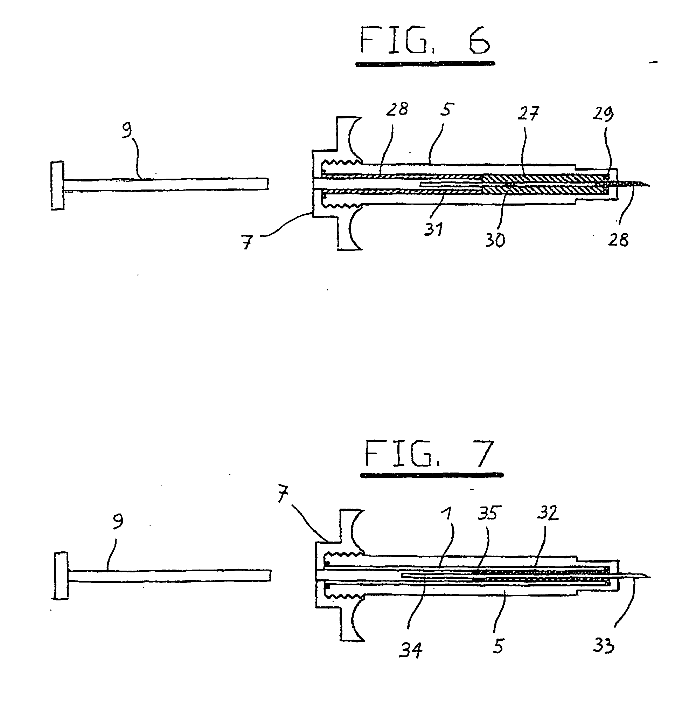

En se référant à la figure 6 on voit une autre forme de réalisation permettant l'injection de volumes réduits. Dans cet exemple le volume interne de l'élément de boîtier 5 est occupé, non pas par un seul tube, mais par un ensemble de deux tubes consécutifs, le premier, à savoir un tube réservoir 27, ayant un très petit diamètre et se prolongeant par l'aiguille 28 avec son embase 29. Un piston de petit diamètre 30 peut se déplacer dans ce tube 27 sous la poussée d'une tige 31 émergeant à l'arrière du tube 27. Le tube 27 est juxtaposé à un second tube 28 ayant le même diamètre externe mais un diamètre interne plus important, dans lequel peut se déplacer la tige de piston 9. La pièce 7 maintient les tubes 25 et 28 en contact l'un avec l'autre et assure la cohésion de l'ensemble comme dans la seringue représentée sur la figure 1.

En se référant à la figure 7 on voit une autre forme de réalisation dans laquelle, pour avoir un volume réduit de formulation en utilisant un tube 1 de grand diamètre, c'est l'embase 32 de l'aiguille 3 qui se prolonge sur une grande distance à l'intérieur du tube 1 pour former le réservoir proprement dit, rempli par la formulation préchargée. Le piston peut être réalisé, par exemple, sous forme d'une tige métallique 34 émergeant dans le tube 1 et traversant un septum 35 qui le maintient.

Les formes de réalisation décrites dans les figures 6 et 7 se prêtent aussi particulièrement bien à la réalisation de réservoirs dont le diamètre interne est égal à celui de la lumière de l'aiguille ou peu différent, par exemple légèrement supérieur.

On conçoit également que le réservoir et l'aiguille soient constitués d'une seule pièce tubulaire se terminant en biseau à l'extrémité de l'aiguille et susceptible d'être traversée par une tige formant piston, de petit diamètre. Dans ce cas, de préférence, le piston est agencé de façon qu'en fin de course, c'est-à-dire à la fin de l'injection, il soit parvenu à proximité de l'extrémité antérieure libre de l'aiguille, de façon à ne laisser pratiquement aucun volume de formulation à l'intérieur de l'aiguille.

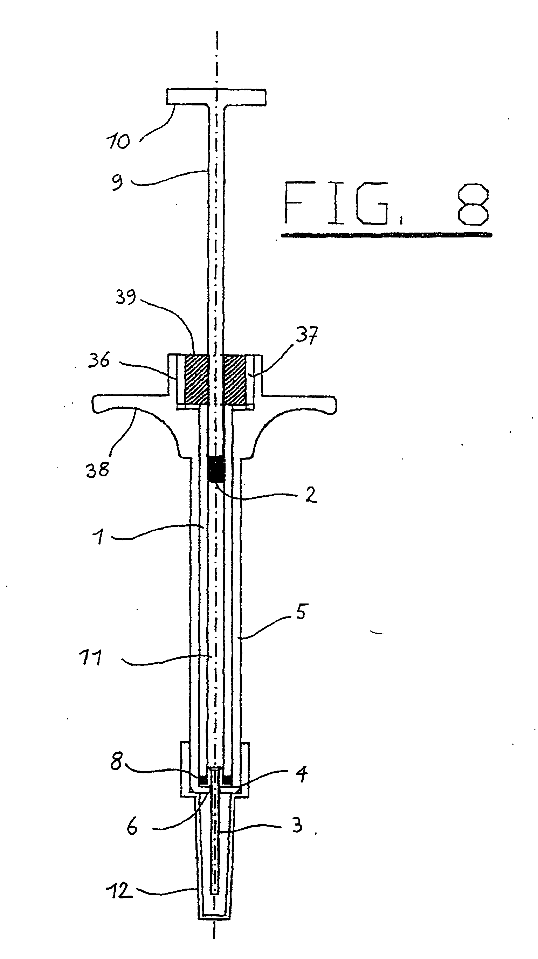

On se réfère à la figure 8 qui représente un exemple de réalisation détaillée d'une seringue selon la figure 1.

Dans cette réalisation, cependant, le deuxième élément de boîtier ne comporte pas une pièce 7. Plus précisément une bague 36 est fixée sur le filetage externe 37 de la pièce 5 et présente les appuie-doigts 38. Le tube 1 est maintenu en position par un bouchon 39 fixé dans l'extrémité de la pièce 5 par vissage ou "clipage".

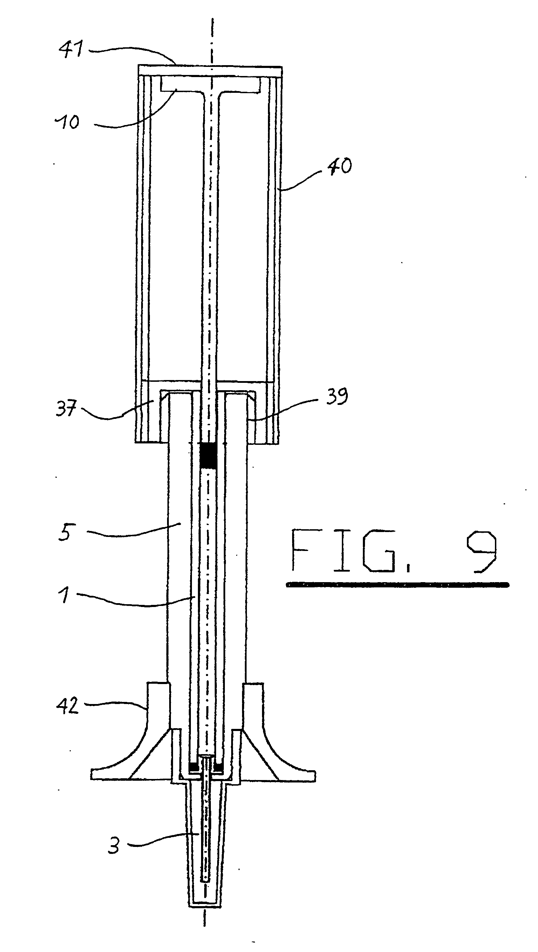

En se référant à la figure 9 on voit un dispositif analogue à celui de la figure précédente mais dans lequel la pièce 36 est supprimée. A la place de la pièce 36 peut venir se visser, sur le filetage 37 de l'élément 5, une pièce en forme de douille 40 intérieurement filetée, dont le fond 41 peut venir s'appliquer contre l'extrémité 10 de la tige de piston 9, que l'on voit dans différentes positions, lorsque l'on visse la douille 40 sur le filetage, de sorte que ce mouvement de vissage provoque l'avancée du piston et l'injection de la formulation semi-solide. Les pas de vis seront réalisés de manière à ce que la durée d'injection ne dépasse pas, par exemple, 30 secondes. A noter que dans ce cas, le joint d'étanchéité est situé entre l'embase 4 et le réservoir 1. Le serrage de l'ensemble doit assurer la compression du joint donc l'asepsie. Vu l'imprécision longitudinale du réservoir (verre) ; un vissage dynamométrique est préféré.

De façon avantageuse un appuie-doigts ou autre moyen de prise 42 peut être prévu, cette fois ci à l'extrémité antérieure du corps 5 pour stabiliser le dispositif durant l'injection.

Pour supprimer les risques d'injection dans un vaisseau, un dispositif d'injection de formulations semi-solides, comprenant un réservoir, une aiguille et un piston, selon l'invention peut être réalisé pour que l'on puisse vérifier la zone d'injection sans avoir à ramener le piston en arrière pour créer une dépression et risquer une cavitation.

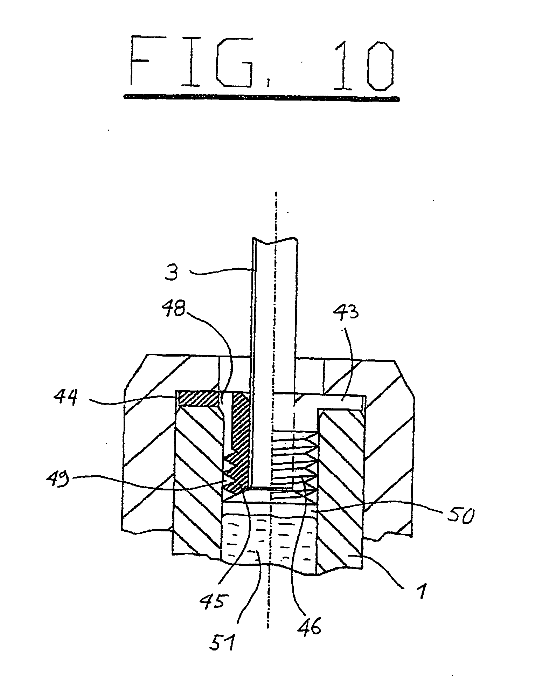

En se référant à la figure 10 on voit une forme de réalisation d'une embase d'aiguille permettant d'atteindre ce résultat.

L'embase 43, qui reçoit l'aiguille 3, présente, comme l'embase 4, une collerette périphérique 44, qui vient prendre appui sur l'extrémité du tube 1, et un prolongement 45, qui pénètre dans le tube, et dont l'extrémité tournée vers le tube transparent 1 présente une forme conique correspondant à la conicité de l'extrémité antérieure du piston permettant d'optimaliser l'écoulement. Ce prolongement 45 présente, à sa surface, un filetage 46 ou une rainure hélicoïdale laissant un volume libre entre elle et la surface intérieure correspondante du tube 1 ou vice-versa. Un perçage 48 met en communication avec l'air libre le volume 49 formé par ce passage hélicoïdal. On comprend que de cette façon l'intérieur du tube 1 communique avec l'air libre par le perçage 48 et le volume hélicoïdal libre 49 délimité par le filetage 46 et qui réaiise un trajet allongé avec une perte de charge contrôlée. La communication avec la lumière interne de l'aiguille 3 peut être assurée, par exemple, en laissant un faible volume libre 50 entre la formulation pâteuse 51 et l'embase 43, ou elle pourrait être assurée par un perçage à travers une partie d'embase mettant en communication l'extrémité interne du filetage 46 avec le volume interne de l'aiguille.

Dès lors que le tube 1 et le corps 5 sont réalisés en matériaux transparents, on comprend que si l'aiguille pénètre dans un vaisseau, de par la pression intra-vasculaire, du sang remontera par la lumière de l'aiguille, en chassant et remplaçant l'air contenu dans le volume 50, le passage hélicoïdal du filetage 46 et éventuellement le volume 47, ce qui permettra de visualiser sur une surface importante l'arrivée du liquide sanguin.

Lorsque la formulation est repoussée par le piston pour être injectée une petite quantité peut pénétrer dans l'interstice hélicoïdal du filetage mais se trouve rapidement bloquée par la perte de charge que représentant la faible section de passage et la grande longueur de cet interstice.

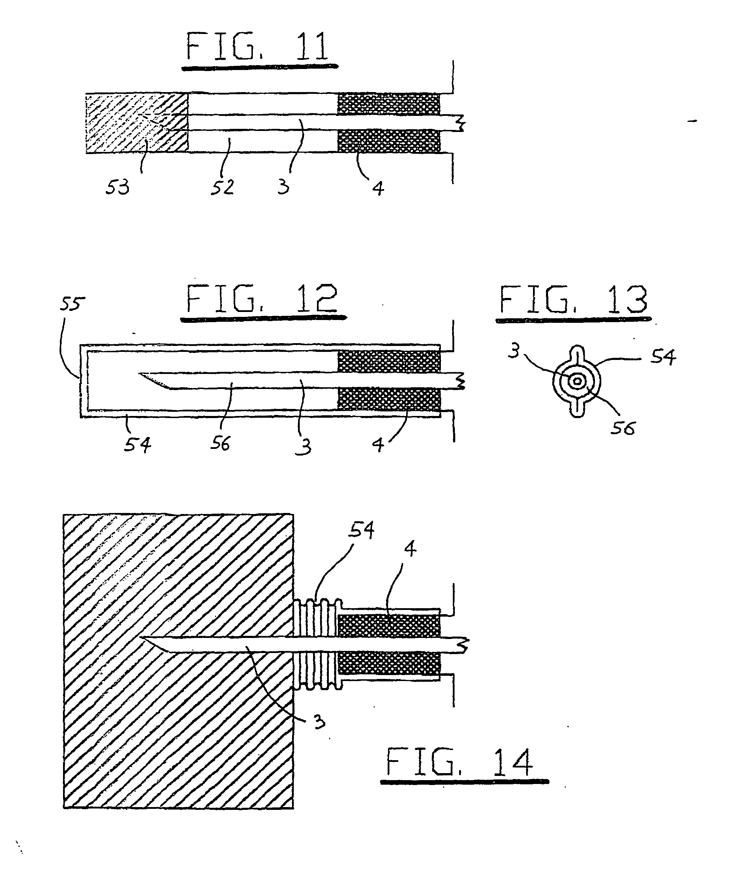

On se réfère maintenant aux figures 11 à 15.

Il est classique, dans le domaine des seringues, d'entourer l'aiguille d'un capuchon amovible qui assure la protection de l'aiguille aussi bien dans l'emballage stérile qu'au moment où la seringue va être utilisée. Cependant la protection de la stérilité de l'aiguille n'existe plus pendant la période séparant la dépose du capuchon de l'injection. On peut résoudre ce problème pour les seringues à formulation semi-solide décrites précédemment, ou pour tout autre type de seringue, en prévoyant, autour de l'aiguille, un emballage flexible fixé, de préférence, d'une façon hermétique, à un élément de la seringue, par exemple l'embase de l'aiguille ou la partie antérieure de l'élément formant réservoir, et susceptible d'être percé par l'aiguille au début de l'injection puis de se rétracter ou se comprimer au fur et à mesure que l'aiguille progresse dans le tissu de la zone d'injection.

De préférence cette enveloppe laisse un espace très faible entre elle et l'aiguille.

De façon particulièrement préférée l'intérieur de l'aiguille et le volume dans lequel elle débouche dans le réservoir de seringue sont maintenus sous vide grâce à la présence de cette enveloppe d'aiguille dont l'intérieur est sous vide.

Ceci permet d'assurer un effet d'aspiration qui, dans le cas où l'aiguille pénètre dans un vaisseau, assure une remontée rapide de liquide sanguin dans l'aiguille puis dans une zone de visualisation conçue pour cela.

En se référant plus particulièrement à la figure 11 on voit un tube fin de plastique 52 qui est scellé par une extrémité à la face intérieure de l'embase 4 et qui entoure l'aiguille 3 à partir de cette face, ce tube étant lui-même scellé dans la zone 53 qui entoure la pointe de l'aiguille.

Dans une variante ce tube 52 peut être retiré pour libérer l'aiguille. Cependant dans une autre variante ce tube peut être conçu de façon à se déformer et se rétracter si l'on appuie la seringue contre l'épiderme du patient pour que son extrémité 53 soit transpercée par l'aiguille.

En se référant plus particulièrement aux figures 12 et 13 on voit qu'à la place d'un tel tube qui entoure étroitement l'aiguille, on peut prévoir un sachet 54 en plastique très fin dont une extrémité entoure de façon hermétique l'embase 4 et qui est obturé à son autre extrémité 55, ce sachet pouvant avoir, par exemple, la forme en section représentée sur la figure 13 laissant un volume très faible 56 entre l'aiguille 3 et le sachet lui-même.

De préférence l'intérieur de la seringue et donc l'intérieur du sachet et le volume 56 est conditionné sous vide.

Lorsque, comme on le voit sur la figure 14, on procède à l'injection, le fond 55 du sachet est transpercé par la pointe de l'aiguille 3 et la progression de l'aiguille provoque la rétraction du sachet 54 appliqué contre l'épiderme. On peut estimer que le passage de l'aiguille à travers le sachet pour pénétrer dans la peau ne devrait pas provoquer de rupture du vide de sorte que si l'aiguille pénètre dans un vaisseau le sang est aspiré à travers l'aiguille et aboutit dans une zone de visualisation, par exemple dans un volume laissé libre entre le débouché de l'aiguille dans le réservoir et la dose de formulation semi-pâteuse ou dans toute autre zone visible en communication avec l'intérieur de l'aiguille, par exemple une zone analogue à la zone 49, mais sans présence d'un trou te! que 48, puisque cette zone ne doit pas être en communication avec l'atmosphère.

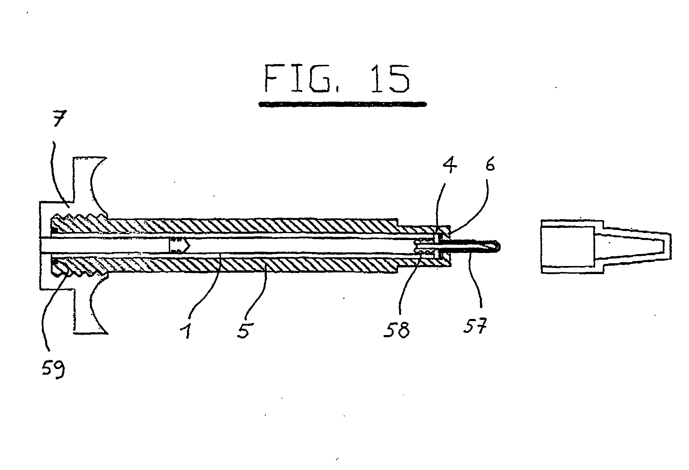

En se référant à la figure 15 on voit schématiquement une seringue selon l'invention dans laquelle un emballage d'aiguille en matière plastique souple, perforable et repliable 57 qui entoure l'aiguille, est serré de façon hermétique contre le fond 6 du corps de seringue 5 par la collerette d'embase 4, cette embase présentant dans la partie qui rentre dans le tube réservoir 1 un filetage 58 analogue au filetage 46 mais ne débouchant pas à l'atmosphère. L'ensemble est maintenu par une pièce de structure 7 avec interposition, autour de l'extrémité postérieure du tube 1, d'un joint d'étanchéité annulaire 59 qui complète l'étanchéité au vide entre le tube 1 et le boîtier 5.

## Revendications

1. Seringue destinée à l'injection parentérale d'une formulation semi-solide, comprenant un élément creux (1) formant réservoir, pour contenir de la préparation semi-solide à injecter entre un piston et une embase (4) d'une aiguille (3) venant en contact avec une extrémité dudit réservoir (1), de façon que le piston (2) parvienne en contact direct avec ladite embase (4) à la fin de l'injection de la dose contenue dans ledit élément formant réservoir, ledit élément formant réservoir (1) et ladite aiguille (3) étant maintenus solidaires l'un de l'autre par application directe de l'élément formant réservoir (1) contre ladite embase par une armature ou boîtier (5,7) recevant ledit élément formant réservoir (1), **caractérisée en ce que** ladite armature ou boîtier (5,7) entoure ledit réservoir avec un jeu, faible ou nul, tel qu'en plus d'assurer le serrage axial de l'élément formant réservoir directement contre ladite embase (4), ladite armature ou boîtier (5,7) renforce le réservoir contre les pressions dues au serrage et à l'administration de la préparation semi-solide.

2. Seringue selon la revendication 1 pré-remplie d'une dose à délivrer entièrement.

3. Seringue selon l'une des revendications 1 à 2, **caractérisée en ce que** l'embase (4) est introduite dans une extrémité du réservoir (1).

4. Seringue selon la revendication 1, **caractérisée en ce que** ledit élément ou réservoir (1), est cylindrique et introduit et verrouillé à l'intérieur d'un corps creux (5) constitué par ladite armature ou boîtier, qui assure la protection et la résistance mécanique, notamment à la pression, de ladite seringue.

5. Seringue selon l'une des revendications 1 à 4, **caractérisée en ce que** ledit réservoir cylindrique (1) est un tube creux rectiligne de diamètres interne et externe constants.

6. Seringue selon la revendication 5, **caractérisée en ce que** le diamètre interne du réservoir (1) est proche ou même égal à celui de la lumière interne de l'aiguille (3) qui prolonge le réservoir.

7. Seringue selon l'une des revendications 1 à 6, **caractérisée en ce que** l'on prévoit vers l'extrémité du réservoir (1) recevant l'aiguille; un rétrécissement régulier conique ou en forme d'entonnoir.

8. Seringue selon l'une des revendications 1 à 7, **caractérisée en ce que** le diamètre interne de l'aiguille (3) est compris entre 0,2 et 1,2 mm, et **en ce que** le diamètre interne du réservoir (1), et donc le diamètre du piston (2) de seringue, est compris entre 0,2 et 5 mm.

9. Seringue selon la revendication 8, **caractérisée en ce que** la course de piston (2) a une longueur maximum égale à 7 cm, pour injecter des volumes de 1 à 10 et jusqu'à 500 µl.

10. Seringue selon l'une des revendications 8 et 9, **caractérisée en ce que** le diamètre externe du réservoir (1) est standardisé, notamment à 6 mm, ce qui permet de prévoir les différents diamètres internes précités.

11. Seringue à réservoir tubulaire selon l'une des revendications 5 à 10, dans laquelle le réservoir (1) est constitué de deux tubes (1, 32) disposés l'un dans l'autre de façon à accroître la résistance à la pression interne.

12. Seringue à réservoir tubulaire selon l'une des revendications 5 à 10, dans laquelle le réservoir tubulaire (1) est constitué de deux ou plusieurs tubes (27, 28) disposés l'un derrière l'autre et maintenus dans cette position par le boîtier, notamment pour faciliter la formation de seringues permettant l'administration de volumes différents.

13. Seringue selon l'une des revendications 1 à 12, **caractérisée en ce que** le boîtier (5, 7) est constitué de deux éléments dont l'un (5) forme un corps creux dans lequel est introduit l'élément formant réservoir et dont l'autre (7) referme le corps creux et emprisonne le réservoir, l'un des éléments (7) laissant une ouverture pour le passage d'une tige de piston (9), et l'autre une ouverture pour le passage de l'aiguille (3).

14. Seringue selon la revendication 13, **caractérisée en ce que** l'ouverture dudit second élément de boîtier (7) forme un guide pour une tige de piston (9) de diamètre sensiblement égal au diamètre interne du réservoir.

15. Seringue selon la revendication 14, **caractérisée en ce que** le second élément (7) comporte des moyens de préhension ou appuie-doigts (8).

16. Seringue selon l'une des revendications 1 à 15, **caractérisée en ce que** des moyens sont agencés, sur ledit premier ou second élément (5, 7), afin de démultiplier la force d'injection ou de remplacer la force manuelle par un moyen d'assistance mécanique ou motrice ou tout autre moyen moteur, notamment à gaz, à ressort ou électromécanique.

17. Seringue selon la revendication 16, **caractérisée en ce que** l'ouverture dans ledit second élément (13) est filetée pour coopérer avec un filetage (15) présenté par la tige de piston pour permettre un déplacement hélicoïdal de ladite tige.

18. Seringue selon la revendication 17, **caractérisée en ce que** ledit second élément (16) présente un filetage périphérique sur lequel on visse une douille intérieurement filetée (17) et présentant une tige de piston centrale (18).

19. Seringue selon l'une des revendications 1 à 18, **caractérisée en ce que** l'assemblage dudit élément formant réservoir (1), avec l'aiguille (3), au niveau d'une embase d'aiguille (4), est réalisé sans collage, ni clipage, ni autre moyen d'assemblage positif, en assurant l'assemblage et la résistance aux forces tendant à désassembler ses pièces, par l'intermédiaire dudit boîtier (5, 7), ledit boîtier étant agencé pour empêcher un écartement axial du réservoir (1) et de l'aiguille (3).

20. Seringue selon l'une des revendications 1 à 19, **caractérisée en ce que** le piston (2), qui peut être solidaire ou non d'une tige de piston (9, 15, 18), présente une forme qui permet de minimiser la résistance à l'écoulement et qui s'adapte à l'embase (4) de l'aiguille ou à l'extrémité du réservoir du côté de l'aiguille de façon à laisser un volume inutilisé aussi faible que possible lorsque le piston (2) est arrivé dans sa position de fin d'injection.

21. Seringue selon l'une des revendications 1 à 20, appartenant à un ensemble de seringues ayant un diamètre constant et une longueur constante du tube réservoir (1) permettant d'utiliser un même boîtier (5, 7) pour des réservoirs prévus pour toute la gamme de doses de formulation.

22. Seringue selon l'une des revendications 1 à 21, **caractérisée en ce que** l'embase (4) de l'aiguille et le piston (2) sont réalisés dans le même matériau, notamment en acier inoxydable.

23. Seringue selon l'une des revendications 1 à 22, **caractérisée en ce que**, pour éviter le risque d'injection dans un vaisseau, la seringue comporte des moyens (46, 47, 48) permettant de vérifier une éventuelle remontée de sang en provenance d'un vaisseau, et ceci sans avoir à tirer sur le piston.

24. Seringue selon la revendication 23, **caractérisée par** une aiguille cathéter permettant une remontée de sang par capillarité jusqu'à une zone ouverte à l'extérieur.

25. Seringue selon l'une des revendications 1 à 24, **caractérisée en ce qu'**elle présente un passage, comprenant une zone visible (46, 49) par l'opérateur, en communication, avec la lumière interne de l'aiguille, permettant de faire apparaître, par pression, capillarité ou dépression, du sang en cas de pénétration de l'aiguille dans une lumière vasculaire.

26. Seringue selon la revendication 25, **caractérisée en ce que**, dans le cas où le sang doit remonter par capillarité, on prévoit que la lumière interne de l'aiguille soit en communication avec l'atmosphère extérieure par un trajet (46, 48, 49) assurant une perte de charge telle qu'un passage de l'air est permis, celui du sang limité, mais que tout passage substantiel de formulation semi-solide ne puisse avoir lieu.

27. Seringue selon l'une des revendications 25 et 26, **caractérisée en ce que** le passage pour le sang depuis l'aiguille (3) passe par le réservoir (1) et comporte un trajet allongé (46) de perte de charge.

28. Seringue selon la revendication 27, **caractérisée en ce que** ledit trajet allongé (46) est compris entre un filetage ou rainure sur l'embase (4) de l'aiguille et une surface complémentaire dans la paroi transparente du réservoir (1), ou réciproquement ce filetage (46) étant en communication, par son extrémité, directement ou par l'intermédiaire d'une zone de visualisation, avec l'atmosphère extérieure par un trou (48) de petit diamètre.

29. Seringue selon la revendication 25, **caractérisée en ce que** l'intérieur de l'aiguille et du réservoir est maintenu sous dépression de sorte qu'une remontée de sang aboutira, par différence de pression, dans une zone de visualisation.

30. Seringue selon la revendication 29, **caractérisée en ce qu'**il comporte une zone de visualisation sans communication avec l'atmosphère.

31. Seringue selon l'une des revendications 1 à 30, **caractérisée en ce que** l'aiguille est recouverte par un capuchon (52), un emballage (54) ou autre protection flexible qui l'isole de l'extérieur et qui sera transpercé par l'aiguille au moment de l'injection, ce-capuchon, emballage ou protection étant rétractable, déformable ou repliable pour s'effacer pendant la pénétration de l'aiguille, et autoriser la pénétration de la totalité ou de la majorité de la longueur de l'aiguille.

32. Seringue selon la revendication 31, **caractérisée en ce que** l'intérieur de la seringue est sous vide.

33. Seringue selon l'une des revendications 31 et 32, **caractérisée en ce que** ledit emballage est constitué d'un tube (52) ou d'un sachet (54) en matière plastique scellé autour de l'aiguille (3) ou sur celle-ci.

34. Seringue selon la revendication 33, **caractérisée en ce que** ledit emballage (52), qui isole complètement l'aiguille (3) de l'extérieur est scellé à l'extrémité de l'aiguille, notamment par thermosoudage, de façon à obturer totalement l'extrémité de l'aiguille à la manière d'un bouchon.

35. Seringue selon la revendication 31 dans laquelle on voit une éventuelle remontée de sang, remontant par capillarité, **caractérisée en ce que** le trou reliant le passage à perte de charge à l'ambiance extérieure débouche, en fait, à l'intérieur de cet emballage, de sorte qu'aucune communication n'existe entre une atmosphère non stérile et l'intérieur de l'aiguille.

36. Seringue selon l'une des revendications 31 à 35, **caractérisée en ce que** ledit capuchon, emballage ou protection est fixée à l'extrémité antérieure du réservoir (1).

37. Seringue selon l'une des revendications 2 à 36, **caractérisée en ce que** le pré-remplissage est tel que le volume de formulation occupe la totalité de l'espace entre le piston et l'aiguille sans qu'il soit nécessaire de purger la seringue avant l'injection.

38. Seringue selon l'une des revendications 1 à 37, **caractérisée en ce que** le piston (2) n'est pas solidaire de la tige de piston et est repoussé par celle-ci en direction d'injection.

39. Seringue selon l'une des revendications 1 à 37, **caractérisée en ce qu'**un joint d'étanchéité (8) est entreposé entre le réservoir (1) et le boîtier (5, 7) pour empêcher une communication avec un interstice situé entre le réservoir et le boîtier (5,7).

40. Procédé de remplissage d'une seringue selon l'une des revendications 1 à 39 dans lequel une buse de remplissage est connectée au niveau dudit tube ou réservoir (1), bouchée par ledit piston (2) ou par un septum et dans lequel ledit piston (2) est déplacé par le remplissage de la formulation, ledit tube étant ensuite bouché par ladite embase (4) de l'aiguille.

41. Procédé de remplissage selon la revendication 40 dans lequel ledit tube est préalablement conditionné dans un emballage et dans lequel il est bouché par introduction dans le corps creux dudit boîtier contenant l'aiguille et porteur du capuchon, le tout à l'intérieur d'un second emballage.

## Patentansprüche

1. Spritze, bestimmt zur parenteralen Injektion einer halbfesten Formulierung, umfassend ein hohles, einen Behälter bildendes Element (1), um ein halbfestes zu injizierendes Präparat zwischen einem Kolben und einem Sockel (4) einer Nadel (3) aufzunehmen, der mit einem Ende des Behälters (1) so in Kontakt tritt, dass der Kolben (2) am Ende der Injektion der Dosis, die in dem den Behälter bildenden Element enthalten ist, in direkten Kontakt mit dem Sockel (4) tritt, wobei das den Behälter bildende Element (1) und die Nadel (3) fest miteinander verbunden gehalten werden durch das direkte Anlegen des den Behälter bildenden Elements (1) an den Sockel durch ein Gestell oder ein Gehäuse (5, 7), welches das den Behälter bildende Element aufnimmt, **dadurch gekennzeichnet, dass** das Gestell oder Gehäuse (5,7) den Behälter mit einem geringen oder keinem Spiel umgibt, so dass zusätzlich zum Sicherstellen des axialen Andrückens des den Behälter ausbildenden Elements gegen den Sockel (4) das Gestell oder Gehäuse (5, 7) den Behälter gegen die Drücke verstärkt, die auf das Andrücken und die Verabreichung des halbfesten Präparats zurückzuführen sind.

2. Spritze nach Anspruch 1, vorab befüllt mit einer vollständig abzugebenden Dosis.

3. Spritze nach einem der Ansprüche 1 bis 2, **dadurch gekennzeichnet, dass** der Sockel (4) in ein Ende des Behälters (1) eingesetzt ist.

4. Spritze nach Anspruch 1, **dadurch gekennzeichnet, dass** das Element oder der Behälter (1) zylindrisch ist und in das Innere eines hohlen Körpers (5) eingesetzt und dort verriegelt wird, der aus dem Gestell oder Gehäuse ausgebildet wird, das den Schutz und die mechanische Widerstandsfestigkeit der Spritze, insbesondere gegen Druck, sicherstellt.

5. Spritze nach einem der Ansprüche 1 bis 4, **dadurch gekennzeichnet, dass** der zylindrische Behälter (1) eine geradlinige hohle Röhre mit konstantem Innen- und Außendurchmesser ist.

6. Spritze nach Anspruch 5, **dadurch gekennzeichnet, dass** der Innendurchmesser des Behälters (1) angrenzend an den oder sogar gleich demjenigen des inneren Lumens der Nadel (3) ist, die den Behälter verlängert.

7. Spritze nach einem der Ansprüche 1 bis 6, **dadurch gekennzeichnet, dass** in Richtung auf das Ende des Behälters (1), das die Nadel aufnimmt, eine regelmäßige konische oder trichterförmige Verjüngung vorgesehen ist.

8. Spritze nach einem der Ansprüche 1 bis 7, **dadurch gekennzeichnet, dass** der Innendurchmesser der Nadel (4) zwischen 0,2 und 1,2 mm beträgt, und dass der Innendurchmesser des Behälters (1) und damit der Durchmesser des Spritzenkolbens (2) zwischen 0,2 und 5 mm beträgt.

9. Spritze nach Anspruch 8, **dadurch gekennzeichnet, dass** der Hub des Kolbens (2) eine maximale Länge von 7 cm hat, um Volumina von 1 bis 10 und bis zu 500 µl zu injizieren.

10. Spritze nach einem der Ansprüche 8 und 9, **dadurch gekennzeichnet, dass** der Außendurchmesser des Behälters (1) genormt ist, insbesondere auf 6 mm, wodurch die vorher erwähnten verschiedenen Innendurchmesser vorgesehen werden können.

11. Spritze mit röhrenförmigem Behälter nach einem der Ansprüche 5 bis 10, in welcher der Behälter aus zwei Röhren (1, 32) gebildet ist, die ineinander angeordnet sind, um so die Widerstandsfestigkeit gegen den Innendruck zu erhöhen.

12. Spritze mit röhrenförmigem Behälter nach einem der Ansprüche 5 bis 10, in welcher der röhrenförmige Behälter (1) aus zwei oder mehreren Röhren (27, 28) gebildet ist, die hintereinander angeordnet sind und in dieser Position durch das Gehäuse festgehalten werden, insbesondere, um das Bilden von Spritzen zu erleichtern, die das Verabreichen von verschiedenen Volumina gestatten.

13. Spritze nach einem der Ansprüche 1 bis 12, **dadurch gekennzeichnet, dass** das Gehäuse (5, 7) aus zwei Elementen gebildet wird, von denen das eine (5) einen hohlen Körper ausbildet, in den das den Behälter bildende Element eingesetzt wird, und von denen das andere (7) den hohlen Körper verschließt und den Behälter umschließt, wobei eines der Elemente (7) eine Öffnung für die Durchführung einer Kolbenstange (9) frei lässt und das andere eine Öffnung für die Durchführung der Nadel (3).

14. Spritze nach Anspruch 13, **dadurch gekennzeichnet, dass** die Öffnung des zweiten Gehäuseelements (7) eine Führung für eine Kolbenstange (9) mit einem Durchmesser ausbildet, der im Wesentlichen gleich dem Innendurchmesser des Behälters ist.

15. Spritze nach Anspruch 14, **dadurch gekennzeichnet, dass** das zweite Element (7) Mittel zum Greifen oder Aufstützvorrichtungen für Finger (8) umfasst.

16. Spritze nach einem der Ansprüche 1 bis 15, **dadurch gekennzeichnet, dass** Mittel auf dem ersten oder zweiten Element (5, 7) angeordnet sind, um die Injektionskraft zu untersetzen oder die manuelle Kraft durch ein mechanisches oder motorisiertes Hilfsmittel oder jedes andere Motormittel zu ersetzen, insbesondere gas-, federoder elektromechanisch betrieben.

17. Spritze nach Anspruch 16, **dadurch gekennzeichnet, dass** die Öffnung in dem zweiten Element (13) mit einem Gewinde versehen ist, um mit einem Gewindegang (15) zusammenzuwirken, den die Kolbenstange aufweist, um eine spiralförmige Verschiebung der Stange zu ermöglichen.

18. Spritze nach Anspruch 17, **dadurch gekennzeichnet, dass** das zweite Element (16) einen peripheren Gewindegang aufweist, auf den eine mit einem Innengewinde versehene Hülse (17) aufgeschraubt werden kann, die eine zentrale Kolbenstange (18) aufweist.

19. Spritze nach einem der Ansprüche 1 bis 18, **dadurch gekennzeichnet, dass** das Zusammenbauen des den Behälter bildenden Elements (1) mit der Nadel (3) auf der Ebene eines Nadelsockels (4) ohne Kleben oder Klemmen oder ein anderes positives Zusammenbaumittel ausgeführt wird, wobei der Zusammenbau und die Widerstandsfähigkeit gegenüber den Kräften, die dazu tendieren, diese Teile auseinander zu bringen, über das Gehäuse (5, 7) sichergestellt werden, wobei das Gehäuse so angeordnet ist, dass eine axiale Beabstandung des Behälters (1) und der Nadel (3) verhindert wird.

20. Spritze nach einem der Ansprüche 1 bis 19, **dadurch gekennzeichnet, dass** der Kolben (2), der mit einer Kolbenstange (9, 15, 18) einstückig sein kann oder nicht, eine Form aufweist, die es gestattet, die Widerstandsfähigkeit gegenüber dem Abfließen zu minimieren, und die sich an den Sockel (4) der Nadel oder an das Ende des Behälters neben der Nadel so anpasst, dass ein kleinstmögliches, nicht verwendetes Volumen übriggelassen wird, wenn der Kolben (2) an seiner Injektionsende-Position angekommen ist.

21. Spritze nach einem der Ansprüche 1 bis 20, zugehörig zu einer Einheit von Spritzen, die einen konstanten Durchmesser und eine konstante Länge des Röhrenbehälters (1) aufweist, wodurch es möglich ist, ein gleiches Gehäuse (5, 7) für Behälter zu verwenden, die für die gesamte Bandbreite von Formulierungsdosierungen vorgesehen sind.

22. Spritze nach einem der Ansprüche 1 bis 21, **dadurch gekennzeichnet, dass** der Sockel (4) der Nadel und der Kolben (2) aus dem gleichen Material ausgeführt sind, insbesondere aus nichtrostendem Stahl.

23. Spritze nach einem der Ansprüche 1 bis 22, **dadurch gekennzeichnet, dass**, um das Risiko einer Injektion in ein Gefäß zu vermeiden, die Spritze Mittel (46, 47, 48) umfasst, die das Überprüfen eines eventuellen Aufsteigens von Blut aus einem Gefäß gestatten, und zwar ohne den Kolben zurückzuziehen.

24. Spritze nach Anspruch 23, **gekennzeichnet durch** einen Nadelkatheter, der ein **durch** Kapillarität bedingtes Aufsteigen von Blut bis zu einem nach außen offenen Bereich gestattet.

25. Spritze nach einem der Ansprüche 1 bis 24, **dadurch gekennzeichnet, dass** sie einen Durchgang aufweist, der einen durch die Bedienperson einsehbaren Bereich (46, 49) umfasst, der mit dem Innenlumen der Nadel in Verbindung steht, der es gestattet, durch Druck, Kapillarität oder Unterdruck auftretendes Blut im Fall des Eindringens der Nadel in ein Gefäßlumen zu sehen.

26. Spritze nach Anspruch 25, **dadurch gekennzeichnet, dass** für den Fall, dass das Blut durch Kapillarität aufsteigen muss, vorgesehen ist, dass das Innenlumen der Nadel mit der Umgebungsatmosphäre über einen Weg (46, 48, 49) in Verbindung steht, der einen Druckverlust so sicherstellt, dass ein Durchlass von Luft gestattet ist, derjenige von Blut begrenzt ist, aber jeder wesentliche Durchlass einer halbfesten Formulierung nicht stattfinden kann.

27. Spritze nach einem der Ansprüche 25 und 26, **dadurch gekennzeichnet, dass** der Durchlass für das Blut von der Nadel (3) aus durch den Behälter (1) führt und einen verlängerten Weg (46) zum Druckverlust umfasst.

28. Spritze nach Anspruch 27, **dadurch gekennzeichnet, dass** der verlängerte Weg (46) zwischen einem Gewindegang oder einer Nut auf dem Sockel (4) der Nadel und einer komplementären Oberfläche in der durchsichtigen Wand des Behälters (1) enthalten ist, oder dieser Gewindegang (46) umgekehrt an seinem Ende direkt oder über einen Sichtbereich mit der Umgebungsatmosphäre durch ein Loch (48) mit kleinem Durchmesser in Verbindung steht.

29. Spritze nach Anspruch 25, **dadurch gekennzeichnet, dass** das Innere der Nadel und des Behälters so unter Unterdruck gehalten wird, dass ein Aufsteigen von Blut durch den Druckunterschied in einen Sichtbereich mündet.

30. Spritze nach Anspruch 29, **dadurch gekennzeichnet, dass** sie einen Sichtbereich ohne Verbindung mit der Atmosphäre umfasst.

31. Spritze nach einem der Ansprüche 1 bis 30, **dadurch gekennzeichnet, dass** die Nadel von einer Kappe (52), einer Verpackung (54) oder einer anderen biegsamen Schutzvorrichtung bedeckt ist, die sie von der Umgebung isoliert und die von der Nadel im Augenblick der Injektion durchstoßen wird, wobei diese Kappe, Verpackung oder Schutzvorrichtung abziehbar, verformbar oder zurückziehbar ist, um während des Eindringens der Nadel zu verschwinden und das Eindringen der Gesamtheit oder des größten Teils der Länge der Nadel zu gestatten.

32. Spritze nach Anspruch 31, **dadurch gekennzeichnet, dass** das Innere der Spritze unter Vakuum steht.

33. Spritze nach einem der Ansprüche 31 und 32, **dadurch gekennzeichnet, dass** die Verpackung aus einem Röhrchen (52) oder einem Tütchen (54) aus Kunststoff besteht, das um die Nadel (3) oder auf dieser versiegelt ist.

34. Spritze nach Anspruch 33, **dadurch gekennzeichnet, dass** die Verpackung (52), welche die Nadel (3) vollständig von der Umgebung isoliert, am Ende der Nadel versiegelt ist, insbesondere durch Warmverschweißen, so dass das Ende der Nadel wie mit einem Pfropfen vollständig verschlossen ist.

35. Spritze nach Anspruch 31, in der ein eventuelles Aufsteigen von Blut beobachtet werden kann, das durch Kapillarität bedingt aufsteigt, **dadurch gekennzeichnet, dass** das Loch, das den Druckverlust-Durchgang mit der Außenumgebung verbindet, tatsächlich im Inneren dieser Verpackung einmündet, so dass keinerlei Verbindung zwischen einer nicht sterilen Atmosphäre und dem Inneren der Nadel vorhanden ist.

36. Spritze nach einem der Ansprüche 31 bis 35, **dadurch gekennzeichnet, dass** die Kappe, Verpackung oder Schutzvorrichtung am vorderen Ende des Behälters (1) befestigt ist.

37. Spritze nach einem der Ansprüche 2 bis 36, **dadurch gekennzeichnet, dass** das Vorab-Befüllen so erfolgt, dass das Formulierungsvolumen die Gesamtheit des Raums zwischen dem Kolben und der Nadel einnimmt, ohne dass es erforderlich ist, die Spritze vor der Injektion zu entlüften.

38. Spritze nach einem der Ansprüche 1 bis 37, **dadurch gekennzeichnet, dass** der Kolben (2) mit der Kolbenstange nicht fest verbunden ist und von dieser in Injektionsrichtung verschoben wird.

39. Spritze nach einem der Ansprüche 1 bis 37, **dadurch gekennzeichnet, dass** eine Dichtung (8) zwischen dem Behälter (1) und dem Gehäuse (5, 7) eingesetzt ist, um eine Verbindung mit dem Zwischenraum zu verhindern, der zwischen dem Behälter und dem Gehäuse (5, 7) liegt.

40. Verfahren zum Befüllen einer Spritze nach einem der Ansprüche 1 bis 39, bei dem eine Befülldüse auf der Ebene der Röhre bzw. des Behälters (1) angeschlossen wird, der durch den Kolben (2) oder durch ein Septum verschlossen ist, wobei der Kolben (2) während des Einfüllens der Formulierung verschoben wird, wobei die Röhre danach durch den Sockel (4) der Nadel verschlossen wird.

41. Verfahren zum Befüllen nach Anspruch 40, bei dem die Röhre vorher in eine Verpackung verpackt wird, in welcher sie durch Einsetzen in den hohlen Körper des Gehäuses verschlossen wird, das die Nadel und den Träger der Kappe enthält, wobei sich alles im Inneren einer zweiten Verpackung befindet.

## Claims

1. A syringe for parenteral injection of a semi-solid formulation, comprising a hollow element (1) forming a reservoir for containing the semi-solid product to be injected between a plunger and a seat (4) of a needle (3) coming into contact with an end of said reservoir (1), such that the plunger (2) comes into direct contact with said seat (4) when the dose contained in said reservoir-forming element. has been injected, said element forming a reservoir (1) and said needle (3) being maintained mutually interlocked by direct application of the element forming a reservoir (1) against said seat by a mount or casing (5, 7) receiving said element forming a reservoir (1), **characterised in that** said mount or casing (5, 7) surrounds said reservoir with a slight or zero clearance such that in addition to effecting the axial clamping of the element forming a reservoir directly against said seat (4), said mount or casing (5, 7) reinforces the reservoir against the pressures due to clamping and to the administration of the semi-solid product.

2. A syringe according to claim 1, pre-filled with a dose to be delivered as a whole.

3. A syringe according to claim 1 or claim 2, **characterised in that** the seat (4) is introduced into an end of the reservoir (1).

4. A syringe according to claim 1, **characterised in that** said element or reservoir (1) is cylindrical and is introduced and locked inside a hollow body (5) constituted by said mount or casing, which provides the protection and the mechanical resistance, especially to pressure, of said syringe.

5. A syringe according .to any one of claims 1 to 4, **characterised in that** said cylindrical reservoir (1) is a straight hollow tube of constant inside and outside diameters.

6. A syringe according to claim 5, **characterised in that** the insi-de diameter of the reservoir (1) is close to or even equal to that of the internal cavity of the needle (3) which extends the reservoir.

7. A syringe according to any one of claims 1 to 6, **characterised in that**, towards the end of the reservoir (1) receiving the needle, a regular conical or funnel-shaped narrowing is provided.

8. A syringe according to any one of claims 1 to 7, **characterised in that** the inside diameter of the needle (3) is between 0.2 and 1.2 mm, and **in that** the inside diameter of the reservoir (1), and therefore the diameter of the syringe plunger (2), is between 0.2 and 5 mm.

9. A syringe according to claim 8, **characterised in that** the stroke of the plunger (2) has a maximum length of 7 cm, for injecting volumes of from 1 to 10 and up to 500 µl.

10. A syringe according to claim 8 or claim 9, **characterised in that** the outside diameter of the reservoir (1) is standardised, especially at 6 mm, thereby making it possible to provide the aforesaid different inside diameters.

11. A syringe with tubular reservoir according to any one of claims 5 to 10, wherein the reservoir (1) is composed of two tubes (1, 32) disposed one inside the other so as to increase the resistance to internal pressure.

12. A syringe with tubular reservoir according to any one of claims 5 to 10, wherein the tubular reservoir (1) is composed of two or more tubes (27, 28) disposed one behind the other and maintained in this position by the casing, especially for facilitating the formation of syringes permitting the administration of different volumes.

13. A syringe according to any one of claims 1 to 12, **characterised in that** the casing (5, 7) is composed of two elements, one of which (5) forms a hollow body into which is introduced the element forming a reservoir and the other of which (7) encloses the hollow body and confines the reservoir, one of the elements (7) leaving an opening for the passage of a plunger rod (9), and the other an opening for the passage of the needle (3).

14. A syringe according to claim 13, **characterised in that** the opening of said second casing element (7) forms a guide for a plunger rod (9) having a diameter substantially equal to the inside diameter of the reservoir.

15. A syringe according to claim 14, **characterised in that** the second element (7) includes gripping means or finger rests (8).

16. A syringe according to any one of claims 1 to 15, **characterised in that** means are arranged, on said first or second element (5, 7), in order to reduce the injection force or to replace the manual force by a mechanical or motive assistance means or any other motive means, especially gas, spring or electromechanical means.

17. A syringe according to claim 16, **characterised in that** the opening in said second element (13) is threaded for co-operating with a thread (15) on the plunger rod to permit helical displacement of said rod.

18. A syringe according to claim 17, **characterised in that** said second element (16) has a peripheral thread on to which is screwed an internally threaded bush (17) having a central plunger rod (18).

19. A syringe according to any one of claims 1 to 18, **characterised in that** the assembly of said element forming a reservoir (1), with the needle (3), at a needle seat (4), is produced without adhesive securing, or clipping or other positive assembly means, the assembly and the resistance to the forces tending to disassemble its parts being provided by means of said casing (5, 7), said casing being arranged to prevent axial separation of the reservoir (1) and the needle (3) from each other.

20. A syringe according to any one of claims 1 to 19, **characterised in that** the plunger (2), which may or may not be integral with a plunger rod (9, 15, 18), has a shape which makes it possible to minimise resistance to the flow and which adapts to the seat (4) of the needle or to the end of the reservoir on the needle side so as to leave the smallest possible unused volume when the plunger (2) has reached its end-of-injection position..

21. A syringe according to any one of claims 1 to 20, belonging to an assembly of syringes having a constant diameter and a constant length of the reservoir tube (1) permitting the use of the same casing (5, 7) for reservoirs provided for the entire range of doses of formulation.

22. A syringe according to any one of claims 1 to 21, **characterised in that** the seat (4) of the needle and the plunger (2) are made of the same material, especially stainless steel.

23. A syringe according to any one of claims 1 to 22, **characterised in that**, in order to avoid the risk of injection into a vessel, the syringe includes means (46, 47, 48) making it possible to detect any rise of blood coming from a vessel, without having to pull on the plunger.

24. A syringe according to claim 23, **characterised by** a catheter needle permitting a rise of blood by capillary action as far as a region open to the outside.

25. A syringe according to any one of claims 1 to 24, **characterised in that** it has a passage, comprising an area (46, 49) visible to the operator, communicating with the internal cavity of the needle, enabling blood to appear, by pressure, capillary action or reduced pressure, in the event of penetration of the needle into a vascular cavity.

26. A syringe according to claim 25, **characterised in that**, in the case where the blood is to rise by capillary action, provision is made for the internal cavity of the needle to communicate with the outside atmosphere by a path (46, 48, 49) ensuring a loss of head such that the passage of air is permitted, that of the blood is limited, but any substantial passage of semi-solid formulation cannot take place.

27. A syringe according to claim 25 or claim 26, **characterised in that** the passage for blood from the needle (3) passes through the reservoir (1) and includes an elongate loss of head path (46).

28. A syringe according to claim 27, **characterised in that** said elongate path (46) is contained between a thread or groove on the seat (4) of the needle and a complementary surface in the transparent wall of the reservoir (1), or, conversely, the thread (46) communicating, by its end, directly or via a visualisation area, with the outside atmosphere through a hole (48) of small diameter.

29. A syringe according to claim 25, **characterised in that** the inside of the needle and of the reservoir is maintained under reduced pressure such that a rise of blood, through a pressure difference, will reach a visualisation area.

30. A syringe according to claim 29, **characterised in that** it includes a visualisation area without communication with the atmosphere.

31. A syringe according to any one of claims 1 to 30, **characterised in that** the needle is covered by a cap (52), a wrapping (54) or another flexible protection which isolates it from the outside and which will be pierced through by the needle at the moment of injection, the cap, wrapping or protection being retractable, deformable or capable of being folded back so as to stay back during the penetration of the needle, and allow the penetration of the entirety or of the majority of the length of the needle.

32. A syringe according to claim 31, **characterised in that** the inside of the syringe is under vacuum.

33. A syringe according to claim 31 or claim 32, **characterised in that** said wrapping is composed of a tube (52) or a bag (54) of plastics material sealed around or onto the needle (3).

34. A syringe according to claim 33, **characterised in that** said wrapping (52), which completely isolates the needle (3) from the outside, is sealed at the end of the needle, especially by heat-welding, so as to completely obturate the end of the needle in the manner of a stopper.

35. A syringe according to claim 31, wherein any rise of blood, rising by capillary action, can be seen, **characterised in that** the hole connecting the loss of head passage to the outside atmosphere in fact opens out inside the wrapping, such that there is no communication between a non-sterile atmosphere and the inside of the needle.

36. A syringe according to any one of claims 31 to 35, **characterised in that** said cap, wrapping or protection is fixed to the forward end of the reservoir (1).

37. A syringe according to any one of claims 2 to 36, **characterised,in that** the pre-filling is such that the volume of formulation occupies the entirety of the space between the plunger and the needle without it being necessary to bleed the syringe before the injection.

38. A syringe according to any one of claims 1 to 37, **characterised in that** the plunger (2) is not integral with the plunger rod and is pushed back by the plunger rod in the injection direction.

39. A syringe according to any one of claims 1 to 37, **characterised in that** a seal (8) is inserted between the reservoir (1) and the casing (5, 7) to prevent communication with a gap located between the reservoir and the casing (5, 7).

40. A method for filling a syringe according to any one of claims 1 to 39, wherein a filling nozzle is connected at said tube or reservoir (1), obturated by said plunger (2) or by a septum and wherein said plunger (2) is displaced by the filling of the formulation, said tube then being obturated by said seat (4) of the needle.

41. A filling method according to claim 40, wherein said tube is previously packaged in a wrapping and wherein it is obturated by introduction into the hollow body of said casing containing the needle and bearing the cap, the whole inside a second wrapping.
